# FASCICULE DE BREVET EUROPEEN

(11) **EP 2 579 932 B1**
(45) Date de publication et mention de la délivrance du brevet: **21.05.2014**
(21) Numéro de dépôt: 11735467.0
(22) Date de dépôt: 09.06.2011
(51) Int. Cl.: A61M 15/00

(54) **DISPOSITIF DE DISTRIBUTION DE PRODUIT FLUIDE**
SPENDEVORRICHTUNG FÜR EIN FLUIDES PRODUKT
DEVICE FOR DISPENSING A FLUID PRODUCT

(30) Priorité: 11.06.2010 FR 1054626
(43) Date de publication de la demande: 17.04.2013
(73) Titulaire: Aptar France SAS, 27110 Le Neubourg (FR)
(72) Inventeur: COLOMB, Arnaud, F-78480 Verneuil sur Seine (FR); SALLAK, Zakaria, F-76100 Rouen (FR)
(74) Mandataire: CAPRI
(86) Numéro de dépôt international: PCT/FR2011/051311
(87) Numéro de publication internationale: WO 2011/154659

(56) Documents cités:
- WO-A1-2009/077697
- WO-A2-2008/012456

## Description

La présente invention concerne un dispositif de distribution de produit fluide, et plus particulièrement un inhalateur de poudre sèche.

Les inhalateurs de poudre sèche sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Un autre type d'inhalateur consiste à emballer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blister allongé ou des blisters disposés sur un disque circulaire rotatif. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre évidemment, l'utilisation est plus complexe, puisque l'utilisateur est obligé de charger une capsule dans l'inhalateur avant chaque utilisation. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a changé la dose (soit à partir d'un réservoir multidoses soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Concernant l'ouverture des réservoirs individuels, il a été proposé de peler ou décoller la couche de fermeture. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. En variante, il a été proposé de percer la paroi ou couche de fermeture. Ceci peut présenter l'inconvénient que les parties de paroi découpées risquent de retenir une partie de la dose à l'intérieur du réservoir, la précision et reproductibilité de dosage n'étant donc pas garanties. Par ailleurs, il est connu d'utiliser un compteur ou indicateur de doses pour informer l'utilisateur du nombre de doses distribuées ou restant à distribuer. Un inconvénient classique avec ces compteurs est que soit ils sont très encombrants, augmentant d'autant la dimension de l'inhalateur lui-même, soit l'affichage est très petit et souvent difficile à lire, notamment pour les personnes âgées. Ceci est notamment vrai pour les compteurs destinés à compter un nombre quelconque de doses, par exemple 30 ou 60 doses. Par ailleurs, pour optimiser l'affichage, les dimensions de la fenêtre de visualisation des compteurs sont généralement proches de celles des nombres à afficher. Il s'en suit que si le nombre à afficher n'est pas parfaitement centré par rapport à ladite fenêtre, cet affichage ne sera pas satisfaisant. Le document WO 2009/077697 décrit un compteur pour inhalateur auquel la présente invention peut s'appliquer.

La présente invention a pour but de fournir un dispositif de distribution de produit fluide, en particulier un inhalateur de poudre sèche qui ne reproduit pas les inconvénients susmentionnés.

En particulier, la présente invention a pour but de fournir un dispositif qui soit simple et peu coûteux à fabriquer et à assembler, fiable d'utilisation, garantissant une précision de dosage et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a encore pour but de fournir un dispositif permettant de compter le nombre de doses émises ou restant à émettre qui soit de dimensions raisonnables tout en proposant un affichage fiable et parfaitement lisible par les utilisateurs après chaque actionnement.

La présente invention a donc pour objet un dispositif de distribution de produit fluide, comportant un corps pourvu d'un orifice de distribution, une pluralité de réservoirs individuels contenant chacun une dose de poudre, des moyens de distribution pour distribuer une dose de poudre à chaque actionnement, lesdits moyens de distribution comportant des moyens d'ouverture de réservoir pour ouvrir un réservoir respectif à chaque actionnement et libérer la dose de poudre, et un compteur de doses pour indiquer le nombre de doses distribuées ou restant à distribuer, ledit compteur comportant au moins un élément de comptage rotatif pourvu de moyens d'indication, tels que des chiffres ou des nombres, et pourvu d'une première denture et d'une seconde denture, un élément d'actionnement mobile, adapté à coopérer avec ladite première denture dudit élément de comptage pour le faire tourner, et des moyens anti-retour adaptés à coopérer avec ladite seconde denture pour empêcher ledit élément de comptage de tourner en sens inverse à celui imparti par ledit élément d'actionnement, caractérisé en ce que lesdits moyens anti-retour comportent une pointe sensiblement en forme de V coopérant après chaque actionnement du compteur avec une dent respective de ladite seconde denture dans une position de blocage, ladite pointe comportant une première branche adaptée à coopérer en position de blocage avec un épaulement de blocage de ladite dent et une seconde branche plane adaptée à coopérer avec une surface plane de ladite dent, ladite seconde branche plane et ladite surface plane étant coplanaires lorsque ladite pointe est en position de blocage, ladite pointe étant sollicitée élastiquement vers ladite position de blocage, pour assurer un positionnement précis dudit élément de comptage après chaque actionnement du compteur.

Avantageusement, lesdits moyens anti-retour sont sensiblement en forme de S, avec une première boucle fournissant l'élasticité et une seconde boucle formant la pointe.

Avantageusement, les deux extrémités du S sont reliées à une partie fixe dudit corps.

Avantageusement, à chaque actionnement du compteur, ledit élément d'actionnement assure une majeure partie de la rotation de l'élément de comptage, ladite pointe assurant la partie finale de ladite rotation.

Ces caractéristiques et avantages et d'autres de la présente invention apparaîtront plus clairement au cours de la description détaillée suivante de plusieurs modes et variantes de réalisation de celle-ci, faite en référence aux dessins joints, donnés à titres d'exemples non limitatifs, sur lesquels,
- la figure 1 est une vue schématique partielle en section d'un dispositif de distribution de produit fluide selon un mode de réalisation avantageux de la présente invention,
- la figure 2 est une vue de détail des moyens anti-retour de la figure 1, avant et après actionnement du compteur,
- la figure 3 est une similaire à celle de la figure 2, pendant l'actionnement du compteur, et
- las figure 4 à 6 sont des vues schématiques de l'affichage du compteur, respectivement avant, pendant et après un actionnement du compteur.

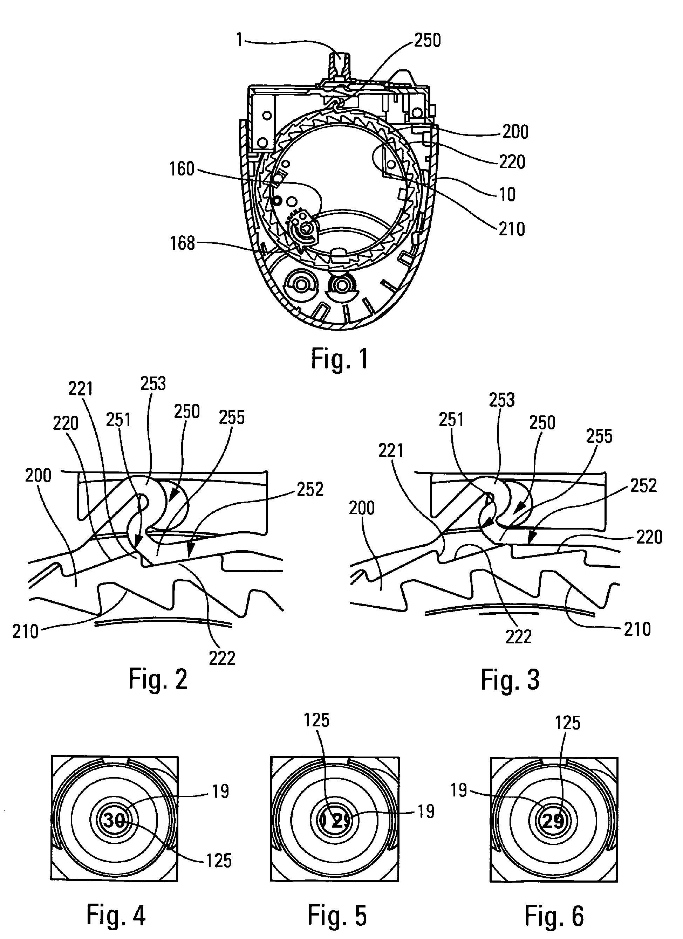

Sur la figure 1 est représenté un exemple avantageux d'un inhalateur de poudre sèche. Cet inhalateur comporte un corps 10 sur lequel peuvent être montées coulissantes deux parties formant capot (non représentées) adaptées à être ouvertes pour ouvrir et charger le dispositif. Le corps 10 peut être de forme environ arrondie comme représenté sur les figures, mais il pourrait avoir tout autre forme appropriée. Le corps 10 comporte un embout buccal ou d'inhalation 1 définissant un orifice de distribution à travers lequel l'utilisateur va inhaler lors de l'actionnement du dispositif. Les capots peuvent s'ouvrir par pivotement autour d'un axe de rotation commun, mais tout autre moyen d'ouverture du dispositif est envisageable. En variante, le dispositif pourrait comporter un seul capot au lieu de deux.

A l'intérieur du corps 10, il est de préférence prévu une bande (non représentée) de réservoirs individuels, également appelés blisters, réalisée sous forme d'une bande allongée sur laquelle les blisters sont disposés les uns derrière les autres, de manière connue. Ces blisters, contenant de préférence de la poudre, ne sont pas représentés sur la figure 1 pour ne pas surcharger le dessin dans des buts de clarté. Cette bande de blister est avantageusement constituée d'une couche ou paroi de base formant les cavités recevant les doses de poudre, et d'une couche ou paroi de fermeture qui obture de manière étanche chacun desdits blisters. Avant la première utilisation, la bande de blister peut être enroulée à l'intérieur du corps 10, de préférence dans une partie de stockage, et des premiers moyens de déplacement de bande (non représentés), notamment rotatifs, sont prévus pour progressivement dérouler et faire avancer cette bande de blister. Des seconds moyens de déplacement, notamment pivotants sur le corps 10, sont prévus pour amener un réservoir individuel ou blister respectif dans une position de distribution à chaque actionnement du dispositif. La partie de bande comportant les réservoirs vides est avantageusement adaptée à s'enrouler dans un autre endroit dudit corps 10, de préférence une partie de réception.

L'inhalateur comporte des moyens d'ouverture de réservoir (non représentés) comportant de préférence des moyens de perçage et/ou de coupage de la couche de fermeture des blisters. Par exemple, les moyens d'ouverture de réservoir comportent avantageusement une aiguille, de préférence fixe par rapport au corps 10, et contre laquelle un blister respectif est déplacé à chaque actionnement par les seconds moyens de déplacement. Le blister est alors percé par ladite aiguille, qui pénètre dans ledit blister pour expulser la poudre au moyen du flux d'inhalation de l'utilisateur.

Les premiers moyens de déplacement sont adaptés à faire avancer la bande de blisters avant et/ou pendant et/ou après chaque actionnement du dispositif. Les seconds moyens de déplacement sont adaptés à déplacer le réservoir à vider contre lesdits moyens de perçage et/ou de coupage lors de l'actionnement. Ces seconds moyens de déplacement peuvent être sollicités par un élément élastique, tel qu'un ressort ou tout autre élément élastique équivalent, ledit élément élastique pouvant être préchargé lors de l'ouverture du dispositif. De préférence, les premiers moyens de déplacement comportent une roue d'indexage qui reçoit et guide les blisters. Une rotation de cette roue fait avancer la bande de blister. Dans une position angulaire particulière, un réservoir donné est toujours en position face aux moyens d'ouverture. Les seconds moyens de déplacement peuvent comporter un élément de support pivotant autour d'un axe de rotation, ladite roue d'indexage étant montée rotative sur ledit élément de support.

Un cycle d'actionnement du dispositif peut être le suivant. Lors de l'ouverture du dispositif, les deux parties latérales formant capot sont écartées l'une de l'autre en pivotant sur le corps pour ouvrir le dispositif, et ainsi charger le dispositif. Dans cette position la roue d'indexage ne peut pas se déplacer vers l'aiguille car les seconds moyens de déplacement sont retenus par des moyens de blocage appropriés. De préférence, c'est lors de l'inhalation par l'utilisateur à travers l'embout buccal 1 que ces moyens de blocage sont débloqués, ce qui provoque alors le déplacement de ladite roue d'indexage en direction de l'aiguille, et donc l'ouverture d'un réservoir.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture de réservoir, on peut prévoir un système de déclenchement par l'inhalation qui comporte avantageusement une unité déplaçable et/ou déformable sous l'effet de l'inhalation (non représentée), cette unité étant adaptée à libérer les moyens de blocage. Cette unité comprend avantageusement une chambre d'air déformable. L'inhalation de l'utilisateur provoque la déformation de ladite chambre d'air déformable, permettant ainsi de libérer lesdits moyens de blocage et donc de permettre le déplacement des seconds moyens de déplacement, et donc d'un réservoir respectif, vers sa position d'ouverture. Le réservoir n'est donc ouvert qu'au moment de l'inhalation, de sorte qu'il est simultanément vidé. Il n'y a donc aucun risque de perte de dose entre l'ouverture du réservoir et son vidage.

D'autres moyens de déclenchement par l'inhalation pourraient aussi être utilisés en variante, par exemple en utilisant un clapet pivotant qui, lorsque l'utilisateur inhale, pivote sous l'effet de la dépression créée par cette inhalation, le pivotement de ce clapet provoquant la libération des moyens de blocage des moyens de support mobiles et donc le déplacement du réservoir vers les moyens d'ouverture.

L'inhalateur comporte en outre une chambre de distribution ou dispersion (non représentée) qui est destinée à recevoir la dose de poudre après ouverture d'un réservoir respectif. Avantageusement, cette chambre de distribution est pourvue d'au moins une bille (non représentée) qui se déplace à l'intérieur de ladite chambre pendant l'inhalation, pour améliorer la distribution du mélange air et poudre après ouverture d'un réservoir, afin d'augmenter l'efficacité du dispositif.

Après l'inhalation, lorsque l'utilisateur referme le dispositif, tous les composants reviennent vers leurs positions de repos initiales. Le dispositif est alors prêt pour un nouveau cycle d'utilisation.

Avantageusement, le corps comporte une fenêtre 19 à travers laquelle le nombre des doses émises ou restant à émettre peut être affiché de manière visible pour l'utilisateur. Cette fenêtre peut par exemple être prévue sur ou proche de l'axe de rotation des éléments de capot formant le capot, mais elle pourrait être à un autre endroit.

Le dispositif selon l'invention comporte un indicateur ou compteur de doses, adapté à compter ou à indiquer à l'utilisateur le nombre de doses distribuées ou restant à distribuer. Cet indicateur comporte au moins un élément de comptage rotatif 200, avantageusement réalisé sous la forme d'un anneau, pourvu d'une première denture 210 et d'une seconde denture 220 et comportant des moyens d'indication 125, par exemple des chiffres ou des nombres, prévus sur une de ses surfaces. De préférence, les moyens d'indication 125 sont disposés sur une surface supérieure, alors que les dentures 210, 220 sont disposées sur une surface inférieure. La première denture 210 est avantageusement adaptée à coopérer avec un élément d'actionnement ou actionneur 160, alors que la seconde denture 220 est avantageusement adaptée à coopérer avec des moyens anti-retour 250, qui sont adaptés à éviter une rotation de l'élément de comptage 200 en sens inverse de celui qui lui est imparti par l'actionneur 160.

Selon l'invention, les moyens anti-retour agissent également en tant que moyens de centrage pour assurer positionnement précis de l'élément de comptage 200, et donc un centrage parfait des moyens d'indication 125 dans la fenêtre 19, après chaque actionnement. Ces moyens anti-retour comportent une pointe 255 sensiblement en forme de V coopérant après chaque actionnement du compteur avec une dent respective de ladite seconde denture 220 dans une position de blocage. Comme visible sur les figures 2 et 3, ladite pointe 255 comporte une première branche 251 adaptée à coopérer en position de blocage avec un épaulement de blocage 221 de ladite dent et une seconde branche plane 252 adaptée à coopérer avec une surface plane 222 de ladite dent. Ladite seconde branche plane 252 et ladite surface plane 222 sont coplanaires lorsque ladite pointe 255 est en position de blocage, et ladite pointe 255 est sollicitée élastiquement vers ladite position de blocage, pour assurer un positionnement précis dudit élément de comptage 200 après chaque actionnement du compteur.

De préférence, lesdits moyens anti-retour sont sensiblement en forme de S. sur les figures 2 et 3, ce S est inversé, mais il pourrait évidemment aussi être réalisé dans l'autre sens. Cette forme de S comporte deux boucles, une première boucle 253 pour assurer l'élasticité, et une seconde boucle formant ladite pointe 255. Avantageusement, les deux extrémités du S sont reliées à une partie fixe du corps 10. Ceci améliore la fiabilité et durée de vie desdits moyens anti-retour. La forme en S est robuste en soi, et le fait que les deux extrémités du S soient fixées au corps élimine le risque de dysfonctionnement même après plusieurs actionnements.

Comme visible sur les figures 2 et 3, la première branche 251 qui coopère avec l'épaulement de blocage 221 de la dent peut être courbe, un seul point de contact suffisant pour assurer le blocage. Au contraire, la seconde branche 252 se conforme à la partie plane 222 de la dent, pour assurer le positionnement angulaire précis de l'élément de comptage 200. Bien entendu, si cette partie 222 de la dent n'était pas exactement plane, la seconde branche 252 ne le serait pas non plus. Ce sont ces deux surfaces en contact coplanaire en position de blocage qui assurent le positionnement précis, contrairement aux moyens anti-retour décrits dans le document WO 2009/077697, ou la pointe de la patte anti-retour ne permet pas de garantir un positionnement angulaire précis après chaque actionnement. Or, comme visible notamment sur les figures 4 à 6, l'affichage des moyens d'indication 125, en l'occurrence les nombres 30 et 29, prend toute la surface de la fenêtre 19. Ainsi, même un léger décalage angulaire risquerait de tronquer le nombre affiché. Au contraire, de par la forme en V de la pointe 255, avec une branche du V en contact coplanaire avec la dent, l'affichage sera toujours précis et centré, comme visible sur les figures 4 et 6.

Lors de l'actionnement du compteur, lorsque l'élément de comptage 200 tourne, la pointe 255 va glisser sur la surface plane 222 de la dent, comme visible sur la figure 5, en déformant élastiquement le S. Lorsque le S passe par-dessus l'épaulement 221 de la prochaine dent, la pointe 255 va s'encliqueter dans cette prochaine dent sous l'effet de l'élasticité du S. Cette même élasticité va solliciter ladite pointe 255 jusqu'à ce que la seconde branche plane 252 affleure la surface plane 222 de la dent. Dans cette position, notamment en raison de l'inclinaison de ladite surface plane 222, la pointe est aussi poussée en contact de l'épaulement 221. Ainsi, après chaque actionnement, la pointe 255 se trouve précisément dans la même position par rapport à la dent avec laquelle elle coopère. Le S étant déformable radialement mais pas angulairement, la position angulaire de l'élément de comptage est donc précisément définie.

Avantageusement, la rotation de l'élément de comptage 200 est assurée en majeure partie, par exemple à au moins 75%, de préférence à environ 90%, par ledit élément d'actionnement 160, le reste étant fourni en fin de rotation par lesdits moyens anti-retour, et notamment ladite pointe 255, sous l'effet de la restitution d'énergie accumulée lors de la déformation élastique dudit S en début de rotation. Ceci évite tout risque de comptage incomplet d'une dose, la rotation angulaire complète de l'élément de comptage étant garantie à chaque dose, d'abord par l'élément d'actionnement et ensuite par les moyens anti-retour.

Avantageusement, le compteur évite de compter des doses qui ne sont pas distribuées, par exemple en cas d'erreur de manipulation, ou d'une manipulation incomplète du dispositif. Il est alors prévu que le compteur ne soir actionné qu'une fois que l'utilisateur a inhalé, puisque c'est cette inhalation qui conditionne l'ouverture et la distribution de la dose de chaque blister. Pour ce faire, le dispositif peut comporter un élément d'actionnement ou actionneur 160 monté pivotant sur le corps 10 et engrené avec les seconds moyens de déplacement. Ainsi, lorsque l'utilisateur ouvre le dispositif et qu'il charge les moyens de chargement du dispositif, les seconds moyens de déplacement ne bougent pas puisqu'ils sont maintenus en position de non-distribution par les moyens de blocage. Il ne se passe donc rien au niveau du compteur puisque l'actionneur 160, monté pivotant sur le corps 10 et engrené avec les seconds moyens de déplacement, reste également immobile. Si l'utilisateur referme le dispositif sans inhaler, il ne se passe évidemment rien non plus puisque les seconds moyens de déplacement restent toujours immobiles. De cette manière, on garantit que le compteur ne compte pas de dose s'il n'y a pas d'inhalation. A partir de la position chargée, si l'utilisateur inhale, il provoque le déplacement des seconds moyens de déplacement dans leur position de distribution en direction des moyens d'ouverture. Ce déplacement provoque donc le pivotement de l'actionneur 160. L'actionneur 160 peut comporter un doigt 168 engrené dans la première denture 210 de l'élément de comptage 200, comme visible sur la figure 1. Dans cette première direction de déplacement, le doigt 168 de l'actionneur peut glisser sur la pente inclinée de la dent correspondante pour venir se positionner face à la prochaine dent. Parallèlement, les moyens anti-retour 250 coopèrent avec la seconde denture 220 de l'élément de comptage 200 pour empêcher une rotation de celui-ci sous l'effet du frottement, exercé par exemple par le doigt 168 de l'actionneur sur la première denture 210. Après l'inhalation, lorsque l'utilisateur referme le dispositif, les seconds moyens de déplacement sont ramenés vers leur position de repos, c'est-à-dire de non-distribution. Ce mouvement provoque donc un pivotement de l'actionneur 160 en sens inverse du précédent. Dans ce déplacement en sens inverse, le doigt 168 de l'actionneur 160 pousse à l'intérieur de la dent dans laquelle il est positionné pour faire tourner l'élément de comptage 200. Parallèlement, les moyens anti-retour 250 glissent sur la pente inclinée 222 de la dent pour venir se positionner dans la dent suivante de la seconde denture 220, en assurant à nouveau son centrage, comme décrit précédemment. En variante, la rotation de l'élément de comptage 200 pourrait avoir lieu lors du déplacement des seconds moyens de déplacement vers leur position de distribution, par exemple en inversant le sens des dents de la première denture 210. En variante également, les première et seconde dentures pourraient être formées par une seule et même denture périphérique, au lieu de deux dentures séparées comme représenté sur la figure 1.

Le compteur peut être adapté à indiquer le nombre de doses restant à distribuer de sorte que le chiffre affiché diminue à chaque actionnement, comme représenté sur les figures 4 à 6. L'inverse est bien entendu également possible, à savoir un compteur qui compte le nombre de doses distribuées. Avantageusement, on peut prévoir des moyens de blocage de l'indicateur après distribution de la dernière dose. Ces moyens de blocage peuvent prendre différentes formes, une forme avantageuse étant de prévoir une dent de forme différente sur la première denture 210 de sorte que l'actionneur ne peut plus venir s'engrener dans la dent suivante pour continuer à faire tourner ledit anneau d'indication. D'autres moyens pour bloquer la rotation du premier anneau après distribution de la dernière dose sont également envisageables.

Le compteur a été décrit avec un élément de comptage, mais il est entendu qu'il peut en comporter au moins un second, comme décrit dans le document WO 2009/077697, pour permettre le comptage d'un plus grand nombre de doses.

L'inhalateur de poudre sèche tel que décrit précédemment procure notamment les fonctions suivantes :
■ une pluralité de doses individuelles de poudre stockées dans des réservoirs individuels étanches, par exemple 30 ou 60 doses stockées sur une bande enroulée en bobine ;
■ la poudre libérée au moyen d'un perçage actionné par l'inhalation de l'utilisateur, ce perçage du blister étant réalisé au moyen d'un système de détection d'inhalation couplé à un système de libération préchargé ;
■ des moyens d'entraînement de forme appropriée en prises avec les blisters pour réaliser le déplacement de la bande de blister à chaque actionnement, et amener un nouveau réservoir dans une position dans laquelle il est destiné à être ouvert par les moyens d'ouverture appropriés ;
■ des moyens pour éviter une perte de dose en cas d'ouverture de l'inhalateur mais en l'absence d'inhalation. Dans ce cas, lors de la fermeture de l'inhalateur, le dispositif revient exactement à sa position de départ ;
■ un indicateur de doses adapté à compter les doses seulement en cas d'inhalation, et garantissant un affichage lisible à chaque actionnement.

D'autres fonctions sont également fournies par l'inhalateur tel que décrit précédemment. Il est à noter que les différentes fonctions, même si elles ont été représentées comme prévues simultanément sur les différents modes de réalisation de l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de l'utilisation d'un indicateur de doses, indépendamment de la manière dont les réservoirs individuels sont arrangés les uns par rapport aux autres, etc. Les moyens d'armement et le système de déclenchement par l'inhalation pourraient être réalisés différemment. Il en est de même des autres parties constitutives du dispositif.

Diverses modifications sont également possibles pour un homme du métier sans sortir du cadre de la présente invention tel que défini par les revendications annexées. En particulier, les diverses caractéristiques et fonctionnalités du dispositif décrites en référence aux différents modes de réalisation et variantes peuvent être adaptées à tous ces modes de réalisation et variantes, et peuvent être combinées entre elles d'une quelconque façon appropriée.

## Revendications

1. Dispositif de distribution de produit fluide, comportant un corps (10) pourvu d'un orifice de distribution (1), une pluralité de réservoirs individuels contenant chacun une dose de poudre, des moyens de distribution pour distribuer une dose de poudre à chaque actionnement, lesdits moyens de distribution comportant des moyens d'ouverture de réservoir pour ouvrir un réservoir respectif à chaque actionnement et libérer la dose de poudre, et un compteur de doses pour indiquer le nombre de doses distribuées ou restant à distribuer, ledit compteur comportant au moins un élément de comptage rotatif (200) pourvu de moyens d'indication (125), tels que des chiffres ou des nombres, et pourvu d'une première denture (210) et d'une seconde denture (220), un élément d'actionnement mobile (160), adapté à coopérer avec ladite première denture (210) dudit élément de comptage (200) pour le faire tourner, et des moyens anti-retour (250) adaptés à coopérer avec ladite seconde denture (220) pour empêcher ledit élément de comptage (200) de tourner en sens inverse à celui imparti par ledit élément d'actionnement (160), **caractérisé en ce que** lesdits moyens anti-retour (250) comportent une pointe (255) sensiblement en forme de V coopérant après chaque actionnement du compteur avec une dent respective de ladite seconde denture (220) dans une position de blocage, ladite pointe (255) comportant une première branche (251) adaptée à coopérer en position de blocage avec un épaulement de blocage (221) de ladite dent et une seconde branche plane (252) adaptée à coopérer avec une surface plane (222) de ladite dent, ladite seconde branche plane (252) et ladite surface plane (222) étant coplanaires lorsque ladite pointe (255) est en position de blocage, ladite pointe (255) étant sollicitée élastiquement vers ladite position de blocage, pour assurer un positionnement précis dudit élément de comptage (200) après chaque actionnement du compteur.

2. Dispositif selon la revendication 1, dans lequel lesdits moyens anti-retour (250) sont sensiblement en forme de S, avec une première boucle (253) fournissant l'élasticité et une seconde boucle formant la pointe (255).

3. Dispositif selon la revendication 2, dans lequel les deux extrémités du S sont reliées à une partie fixe dudit corps (10).

4. Dispositif selon l'une quelconque des revendications précédentes, dans lequel, à chaque actionnement du compteur, ledit élément d'actionnement (160) assure une majeure partie de la rotation de l'élément de comptage (200), ladite pointe (255) assurant la partie finale de ladite rotation.

## Patentansprüche

1. Spendevorrichtung für ein fluides Produkt bestehend aus einem Körper (10) versehen mit einer Spendeöffnung (1), einer Vielzahl an individuellen Tanks, von denen jeder eine Dosis Pulver, Spendemittel zum Spenden einer Dosis Pulver bei jedem Auslösen vefügt, wobei die genannten Spendemittel über Vorrichtungen zur Öffnung eines Tanks verfügen, um den zu jedem Auslösen gehörigen Tank zu öffnen und die Pulverdosis freizugeben, sowie einen Dosenzähler, um die Anzahl der bereits verteilten Dosen oder der noch zu verteilenden Dosen anzuzeigen, wobei dieser Zähler mindestens ein rotierendes Zählelement (200) umfasst, das über Anzeigemittel (125) verfügt wie Zahlen oder Namen und versehen mit einer ersten Vezahnung (210) und einer zweiten Verzahnung (220), einem beweglichen Element zur Auslösung (160), angepasst zur Zusammenarbeit mit der genannten ersten Verzahnung (210) des genannten ersten Zählelement (200), damit dieses sich dreht und Mittel, die den Rückfluss verhindern (250), um zu vermeiden, dass das gesamte Zählelement (200) in die entgegengesetzte Richtung zu der Richtung dreht, die von dem genannten Auslöseelement (160) vorgegeben wird, **dadurch gekennzeichnet, dass** die genannten Mittel, die den Rückfluss verhindern (250) über eine Spitze (255) vorzugsweise in V-Form verfügen, die mit jedem Auslösen des Zählers mit einem entsprechenden Zahn der genannten zweiten Verzahnung (20) in einer Sperrposition zusammenarbeitet, wobei die genannte Spitze (255) einen ersten Bereich (251) umfasst, der dazu angepasst ist, in der Sperrposition mit einem Sperrvorsprung (221) des genannten Zahns zusammenzuarbeiten und einen zweiten flachen Bereich (252), der angepasst ist, um mit einer flachen Oberfläche (222) des genannten Zahns zusammenzuarbeiten, wobei der genannte zweite flache Bereich (252) und die genannte flache Oberfläche (222) koplanar sind, wenn sich die genannte Spitze (255) in der Sperrposition befindet, wobei die genannte Spitze (255) elastisch in Richtung der genannten Sperrposition belastet wird, um eine präzise Positionierung des genannten Zählelements (200) nach jedem Auslösen des Zählers zu gewährleisten.

2. Vorrichtung nach Anspruch 1, wobei die genannten Mittel gegen den Rückfluss (250) vorzugsweise in S-Form sind, mit einem ersten Bogen (253), der Elastizität gibt und einem zweiten Bogen, der die Spitze (255) bildet.

3. Vorrichtung nach Anspruch 2, wobei die zwei Enden des S mit einem festen Teil des genannten Körpers (10) verbunden sind.

4. Vorrichtung nach einem der vorangegangenen Ansprüche, bei dem bei jedem Auslösen des Zählers das genannte Auslöseelement (160) den größten Teil der Drehung des Zählelements (200) gewährleistet und die genannte Spitze (255) den abschließenden Teil der genannten Drehung gewährleistet.

## Claims

1. A fluid dispenser device comprising: a body (10) provided with a dispenser orifice (1); a plurality of individual reservoirs each containing one dose of powder; dispenser means for dispensing one dose of powder on each actuation, said dispenser means including reservoir opening means for opening a respective reservoir on each actuation and for releasing the dose of powder; and a dose counter for indicating the number of doses that have been dispensed or that remain to be dispensed, said counter comprising: at least one rotary counter element (200) provided with indicator means (125), such as digits or numbers, and provided with a first set of teeth (210) and with a second set of teeth (220); a movable actuator element (160) adapted to co-operate with said first set of teeth (210) of said counter element (200) so as to turn it; and anti-return means (250) adapted to co-operate with said second set of teeth (220) so as to prevent said counter element (200) from turning in the direction opposite to the direction that is imposed by said actuator element (160); the device being **characterized in that** said anti-return means (250) comprise a tip (255) that is substantially V-shaped, and that, after each actuation of the counter, co-operates with a respective tooth of said second set of teeth (220) in a blocking position, said tip (255) comprising a first branch (251) that is adapted to co-operate in the blocking position with a blocking shoulder (221) of said tooth, and a second branch (252) that is plane and adapted to co-operate with a plane surface (222) of said teeth, said plane second branch (252) and said plane surface (222) being coplanar when said tip (255) is in the blocking position, said tip (255) being urged resiliently towards said blocking position so as to ensure accurate positioning of said counter element (200) after each actuation of the counter.

2. A device according to claim 1, wherein said anti-return means (250) are substantially S-shaped, with a first loop (253) providing springiness, and a second loop forming the tip (255).

3. A device according to claim 2, wherein the two ends of the S are connected to a stationary portion of said body (10).

4. A device according to any preceding claim, wherein, on each actuation of the counter, said actuator element (160) provides a major portion of the turning of the counter element (200), said tip (255) providing the final portion of said turning.
